(19) Europäisches Patentamt
European Patent Office
Office européen des brevets

(11) Publication number: **0 157 448 A1**

(12) **EUROPEAN PATENT APPLICATION**

(21) Application number: 85200386.2

(22) Date of filing: 15.03.85

(51) Int. Cl.[4]: C 07 C 126/02

(30) Priority: 16.03.84 NL 8400840

(43) Date of publication of application: 09.10.85 Bulletin 85/41

(84) Designated Contracting States: AT BE DE FR GB IT NL

(71) Applicant: UNIE VAN KUNSTMESTFABRIEKEN B.V., Maliebaan 81, NL-3581 CG Utrecht (NL)

(72) Inventor: Van Nassau, Petrus Johannes Marie, Burg. Pijlsstraat 4, NL-6151 HA Munstergeleen (NL)

(74) Representative: Roeffen, Wilhelmus Johannes Maria et al, OCTROOIBUREAU DSM Postbus 9, NL-6160 MA Geleen (NL)

(54) **Process for the preparation of urea.**

(57) A process for the preparation of urea in which an urea synthesis solution containing carbamate and free ammonia is formed from carbon dioxide and an excess of ammonia in a reaction zone at a pressure of 130–250 bar, the carbamate is decomposed in at least two decomposition zones, not isobaric relative to one another and arranged in series, and the decomposition products formed, together with non-converted ammonia, are removed from the solution, in the first decomposition zone a pressure of 130–250 bar being maintained and in the second decomposition zone a lower pressure, after which the urea-containing solution obtained in the second decomposition zone is processed into a carbamate-free urea solution or solid urea. The process is characterized in that in the second decomposition zone a pressure of 85–125 bar is applied and the gas mixture containing ammonia and carbon dioxide that is obtained here is condensed at the pressure of the second decomposition zone in a condensation zone, where also 40–80% of the equilibrium amount of urea achievable under the prevailing conditions is allowed to form, and the carbamate and the urea formed are fed to the reaction zone.

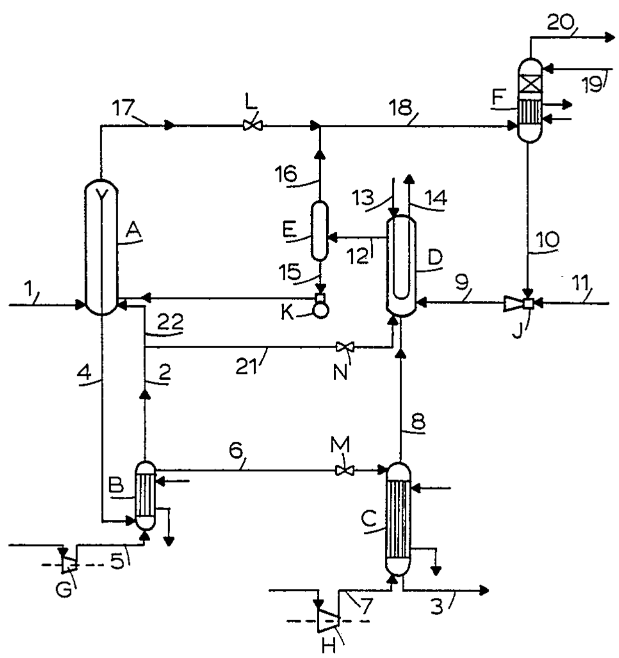


EP 0 157 448 A1

Unie van Kunstmestfabrieken B.V.



# PROCESS FOR THE PREPARATION OF UREA

The invention relates to a process for the preparation of urea from ammonia and carbon dioxide.

If ammonia and carbon dioxide are introduced into a synthesis zone under a suitable pressure (for instance 125-350 atm.) and at a suitable temperature (for instance 170-250 °C), first ammonium carbamate is formed, according to the reaction:

$$2\ NH_3 + CO_2 \rightarrow H_2N\text{-}CO\text{-}ONH_4$$

From the ammonium carbamate thus obtained, subsequently urea is formed by dehydration according to the reversible reaction:

$$H_2N\text{-}CO\text{-}ONH_4 \rightleftharpoons H_2N\text{-}CO\text{-}NH_2 + H_2O$$

The degree to which the conversion to urea takes place depends among others on the temperature and the ammonia excess used. As reaction product, a solution is obtained that consists mainly of urea, water, ammonium carbamate and free ammonia. The ammonium carbamate and the ammonia are to be removed from the solution and are mostly returned to the synthesis zone.

According to a known process (see British patent specification 2,087,381) urea is prepared at a pressure of 180-250 bar in a reaction zone where a molar $NH_3$ : $CO_2$ ratio of 5-8 is maintained, following which the urea synthesis solution formed is subjected to a stripping treatment in two successive decomposition steps. In the first decomposition step, where a pressure is maintained that is virtually equal to the pressure in the synthesis zone and a temperature of between 180 and 215 °C, the excess ammonia present in the solution serves as stripping agent for the carbamate decomposition products. In the second decomposition step, where a pressure of 30-50 bar lower than that of the first decomposition step is maintained and a temperature of between 160 and 210 °C, carbon dioxide is used as stripping agent. The gas mixture containing ammonia and carbon dioxide that is obtained in the first decomposition stage is as such, without further treatment, led to the synthesis zone. The gas mixture containing ammonia and carbon dioxide that is obtained in the second decomposition step is condensed at a

pressure pratically equalling that of the second decomposition step, and the condensate formed is brought up to the synthesis zone pressure by means of a carbamate pump and is recycled to the synthesis zone.

According to another known process (see British patent specification 2,107,311), not only the decomposition of carbamate not converted into urea and the expulsion of the gases thus obtained together with the excess ammonia present is carried out in two steps, but also the urea synthesis, different $NH_3$ : $CO_2$ molar ratios being maintained in the two reaction zones. The pressure of the second reaction zone preferably equals that of the first decomposition zone, and the pressure of the first reaction zone equals that of the second decomposition zone. The modes of realization described in the examples indicate that the pressure of the second decomposition zone is preferred to be 160 bar.

These known processes have the disadvantage that the maximum decomposition efficiency in the second decomposition step cannot be reached since the high temperature required for carbamate decomposition at the high pressure employed necessitates a very short residence time to avoid unacceptable degrees of urea hydrolysis and biuret formation. This not only adversely affects the synthesis efficiency, but also causes the biuret content of the urea synthesis solution produced to increase to a most undesirable extent. Since part of the excess ammonia present is removed already in the first decomposition zone in processes comprising two-step carbamate decomposition by stripping, said side reactions take place to an increased extent in the second decomposition step.

The object of the invention is to provide a process in which a high decomposition efficiency is achieved and in which urea hydrolysis and biuret formation during carbamate decomposition are restricted to a minimum. According to the invention this is accomplished by having the carbamate decomposition in the second step take place at substantially lower pressure, and consequently substantially lower temperature, than in the above-mentioned known processes. To enable recovery at a sufficiently high temperature level of the heat of condensation liberated upon condensation, at the lower temperature attending the lower pressure, of the ammonia- and carbon dioxide-containing gas mixture obtained by carbamate decomposition, so that this heat can be utilized for obtaining useful low-pressure steam, e.g. of 4 bar, according to

the invention urea is also allowed to form in the carbamate condensation zone.

The invention therefore relates to a process for the preparation of urea in which a urea synthesis solution containing carbamate and free ammonia is formed from carbon dioxide and an excess of ammonia in a reaction zone at a pressure of 130-250 bar, the carbamate is decomposed in at least two decomposition zones, not isobaric relative to one another and arranged in series, and the decomposition products formed, together with non-converted ammonia, are removed from the solution, in the first decomposition zone a pressure of 130-250 bar being maintained and in the second decomposition zone a lower pressure, after which the urea-containing solution obtained in the second decomposition zone is processed into a carbamate-free urea solution or solid urea. The process is characterized in that in the second decomposition zone a pressure of 85-125 bar is applied and the gas mixture containing ammonia and carbon dioxide that is obtained here is condensed at the pressure of the second decomposition zone in a condensation zone, where also 40-80% of the equilibrium amount of urea achievable under the prevailing conditions is allowed to form, and the carbamate and the urea formed are fed to the reaction zone.

By preference a pressure of 110-120 bar is applied in the second decomposition zone, because then a considerable amount of carbamate is decomposed and, due to the relatively low temperatures attending these pressures, only slight amounts of urea hydrolyse or are converted into biuret. The use of pressures below 85 bar results in a very high decomposition efficiency being achieved. On condensation of the gases containing ammonia and carbon dioxide obtained in carbamate decomposition, however, the lower condensation temperature will lead to the production of steam with a pressure lower than 3.5 bar, which is difficult to utilize in the process. For this reason, application of pressures below 85 bar is less desirable. The use of pressures in excess of 125 bar demands relatively high temperatures, and in the ammonia-lean environment, with increasing pressure and corresponding temperature, urea hydrolysis and biuret formation increasingly take place.

In the process according to the invention, the heat of carbamate formation is recovered in the condensation zone by having the condensation of the ammonia- and carbon dioxide-containing gases

obtained in the second decomposition step take place in the presence of relatively large amounts of urea and water. These then serve as solvent for carbamate. As a result, upon condensation the heat will become available at a higher temperature level than without the presence of this solvent. The conversion of carbamate into urea and water in the condensation zone is effected by making the residence time of the reaction mixture in the condensation zone sufficiently long. To increase the temperature further, the condensation zone may be designed as a reactor with intensive mixing in which the temperature difference between top and bottom is limited to 5 °C at most. By preference, this temperature difference is kept at 2 °C at most. Condensation may be effected, for instance on the shell side of a vertical tubular heat exchanger. The heat released in the condensation zone may then be removed by means of water led through the tubes and thus converted into low-pressure steam. It is also possible to use other condensation zones having such dimensions as to allow of a sufficiently long residence time of the reaction mixture and provided with a number of coils through which cooling water is led, with the aid of which the heat of condensation is removed. By preference, the condenser takes the form of a so-called submerged condenser, the gas mixture to be condensed being led into a dilute carbamate solution and the heat of dissolution and the heat of condensation released being removed with water, which is thereby converted into steam. By carrying out the condensation of the gas mixture containing ammonia and carbon dioxide in the way described, the temperature in the condensation zone can, depending on the amounts of urea and water formed, be increased by on average 4-10 °C, making it possible to generate low-pressure steam of 3.5-4.5 bar at the condensation zone pressure of 85-125 bar. The amount of urea that is allowed to form may be 40-80% of the amount that can be achieved under the given reaction conditions. By preference, at least 50-60% of the amount of urea achievable under the reaction conditions is allowed to form.

The invention will be elucidated on the basis of a figure and the example, without however being restricted thereto.

In the figure, A indicates a synthesis zone, B represents a first decomposition zone, C a second decomposition zone, D a condensation zone, E a gas-liquid separator and F a washing zone. G and H indicate compressors for compression of air and carbon dioxide,

respectively, J is an ejector and K a carbamate pump. L, M and N are expansion valves.

Through 1 liquid ammonia is supplied to synthesis zone A, through 2 a gas mixture coming from first stripping zone B and containing ammonia and carbon dioxide, and through 15 a solution containing carbamate and urea, formed in condensation zone D and brought at the pressure prevailing in synthesis zone A, from 130 to 250 bar, e.g. 160 bar, by means of pump K. The urea synthesis solution formed flows through 4 into first decomposition zone B, represented in the figure as a steam-heated cocurrent heater-decomposer, in which the urea synthesis solution to be treated and the ammonia- and carbon dioxide-containing gases expelled due to heating flow in the same direction through the heater tubes. Compressor G leads a small amount of air through 5 into first decomposition zone B so as to keep the materials coming into contact with carbamate-containing solutions at high temperatures in passive condition. Through 2 and 22 the expelled gases are led into synthesis zone A, where they react to form carbamate and where the temperature required for the urea reaction is maintained by the heat of carbamate formation released. If desired, part of the gases obtained can be passed through 21 and expansion valve N to condensation zone D. The urea synthesis solution partly freed of carbamate and free ammonia flows through 6 and expansion valve M, where the pressure is reduced to 85-125 bar, e.g. to 110 bar, into second decomposition zone C, which is heated by steam. In second decomposition zone C the supplied solution is stripped with carbon dioxide, supplied through 7 and compressed to the required pressure by compressor H, to which a small amount of air has been added to keep the materiaal in passive condition. The stripped urea synthesis solution is discharged through 3 and processed into a urea solution or solid urea in a known way. The gases expelled in second decomposition zone C flow, together with the carbon dioxide used as stripping gas, through 8 into condensation zone D. Condensation zone D may for instance be a submerged condenser. The condensation zone is further supplied with the carbamate solution obtained upon washing out of the inert gases discharged from the system in washing colomn F; from F this carbamate solution is passe through 10 to ejector J, driven by liquid ammonia supplied through 11, which leads it into condensation zone D through 9. The volume of condensation zone D is designed so that the

residence time of the reaction mixture in this zone is suffciently long, so that in addition here 40-80% of the equilibrium amounts of urea and water achievable under the prevailing conditions are formed from the carbamate present. The heat released is removed with the aid of water, which is supplied through 13 and converted into low-pressure steam of about 4 bar which is removed through 14. The gas-liquid mixture containing urea and carbamate is led through 12 to gas-liquid separator E, from which the liquid phase is passed through 15 via carbamate pump K to synthesis zone A, in which further conversion into urea takes place. The gas phase obtained in gas-liquid separator E is discharged through 16 and led through 18 into washing zone F, together with the gas phase discharged from A through 17, after the pressure of the latter has been reduced to the pressure of gas-liquid separator E in expansion valve L. In washing zone F, the ammonia- and carbon dioxide-containing inert gases are washed out with a carbamate solution supplied through 19, which has been obtained in further processing of the stripped urea synthesis solution into a urea solution or solid urea. The heat of condensation and the heat of absorption released in this washing process are removed, insofar as necessary, by means of cooling water or any other suitable cooling medium. The inert gases are vented through 20.

Example

Urea is prepared by the process described here and according to the mode of realization represented in the figure, in a plant having a production capacity of 1500 tons a day. The amounts are given in kg an hour. The pressure applied in synthesis zone A and first decomposition zone B is 176,5 bar, while that in second decomposition zone C, condensation zone D and washing zone F is 107.9 bar.

Through 1, 13,432 kg liquid $NH_3$ of 33 °C is fed to synthesis zone A, and through 11 21,185 kg is fed to condensation zone D. The $CO_2$ required in the process is led into second decomposition zone C in an amount of 45,833 kg, together with 1,469 kg inert components, mainly air, with a temperature of 120 °C. Into decomposition zone B, in addition, 292 kg passivation air is introduced.

Condensation zone D is supplied with:

a) 32,016 kg carbamate solution, consisting of 13,380 kg $NH_3$, 12,694 kg $CO_2$, 5,942 kg $H_2O$, from washing zone F, together with

21,185 kg fresh, liquid $NH_3$;

b) 95,110 kg gas mixture, consisting of 36,539 kg $NH_3$, 53,802 kg $CO_2$, 3300 kg $H_2O$ and 1,469 kg inerts, from second decomposition zone C;

c) 6,010 kg gas mixture, consisting of 3,616 kg $NH_3$, 2,105 kg $CO_2$, 237 kg $H_2O$ and 52 kg inerts, from decomposition zone B.

The volume of the condensation zone, and thus the residence time, is chosen so that at the selected pressure of 107,9 bar and a temperature of about 164 °C a gas-liquid mixture is obtained, in the liquid phase of which about 50 % of the amount of urea leaving synthesis zone A is formed. This liquid phase is composed of 33,063 kg urea, 48,380 kg $NH_3$, 37,794 kg $CO_2$ and 19,036 kg $H_2O$. The gas phase contains 7,604 kg $NH_3$, 6,561 kg $CO_2$, 362 kg $H_2O$ and 1,521 kg inerts. From the liquid phase obtained, which is brought at a pressure of 176,5 bar by pump K, and the liquid $NH_3$ supplied through 1, in synthesis zone A at a temperature of 190 °C a urea synthesis solution consisting of 66,125 kg urea, 58,292 kg $NH_3$, 22,324 kg $CO_2$ and 29,977 kg $H_2O$ is formed. Through 17, 2,370 kg gas mixture, consisting of 1,259 kg $NH_3$, 815 kg $CO_2$, 56 kg $H_2O$ and 240 kg inerts, is discharged from this zone. The urea synthesis solution formed in synthesis zone A is subjected to a first carbamate decomposition in first-decomposition zone B, which is designed as a steam-heated cocurrent heater decomposer, upon which a portion of the carbamate present decomposes into $NH_3$ and $CO_2$, which are removed together with a portion of the free $NH_3$ and $H_2O$ present in the solution. Through 21, an amount of 6,010 kg of the gas mixture expelled is sent to condensation zone D, after its pressure has been decreased to 107.9 bar. The remaining part, consisting of 16,474 kg $NH_3$, 9,591 kg $CO_2$, 1,078 kg $H_2O$ and 240 kg inerts, is returned to synthesis zone A. The liquid phase remaining in first decomposition zone B, consisting of 66,125 kg urea, 38,202 kg $NH_3$, 10,628 kg $CO_2$ and 28,662 kg $H_2O$, is, after being reduced in pressure to 107,9 bar, led into second decomposition zone C and stripped countercurrent to $CO_2$, heat meanwhile being supplied. From this zone, 95,809 kg stripped urea synthesis solution is obtained, which besides 63,480 kg urea and 24,568 kg $H_2O$, also contains 3,162 kg $NH_3$ and 4,599 kg $CO_2$. The gas mixture obtained in the stripping treatment in second decomposition zone C is led direct into condensation zone D through 8.

In washing zone F, the inerts-containing gas mixtures from

synthesis zone A and condensation zone D are washed out with 15,605 kg carbamate solution, consisting of 4,649 kg $NH_3$, 5,432 kg $CO_2$ and 5,524 kg $H_2O$. The inert gases, 2,007 kg, are vented through 20. In first decomposition zone B 441 kg high-pressure steam of 24.5 bar (265 °C) is needed and in second decomposition zone C 340 kg. In condensation zone D 907 kg low-pressure steam of 4 bar (144 °C) is obtained.

CLAIMS AE 3522

1. Process for the preparation of urea in which a urea synthesis solution containing carbamate and free ammonia is formed from carbon dioxide and an excess of ammonia in a reaction zone at a pressure of 130-250 bar, the carbamate is decomposed in at least two decomposition zones, not isobaric relative to one another and arranged in series, and the decomposition products formed, together with non-converted ammonia, are removed from the solution, in the first decomposition zone a pressure of 130-250 bar and in the second decomposition zone a lower pressure being maintained, after which the urea-containing solution obtained in the second decomposition zone is processed into a carbamate-free urea solution or solid urea, this process being characterized in that a pressure of 85-125 bar is applied in the second decomposition zone and the gas mixture containing ammonia and carbon dioxide that is obtained here is condensed at the pressure of the second decomposition zone in a condensation zone, in which also 40-80% of the equilibrium amount of urea achievable under the prevailing conditions is allowed to form, and the carbamate and the urea formed are supplied to the reaction zone.
2. Process according to claim 1, characterized in that the gas mixture containing ammonia and carbon dioxide is condensed at a pressure of 110-120 bar.
3. Process according to claim 1-2, characterized in that 50-60% of the equilibrium amount of urea achievable under the prevailing conditions is allowed to form.
4. Process according to any one of claims 1-3, characterized in that the carbamate- and urea-containing mixture formed in the condensation zone is subjected to a gas-liquid separation, ammonia and carbon dioxide are recovered from the gas mixture thus separated, the non-condensable part of the gas mixture is discharged and the remaining carbamate- and urea-containing solution is led into the reaction zone.
5. Process for the preparation of urea as described and elucidated on the basis of the drawing and the example.
6. Urea and urea solutions prepared by the process according to one or more of the aforegoing claims.

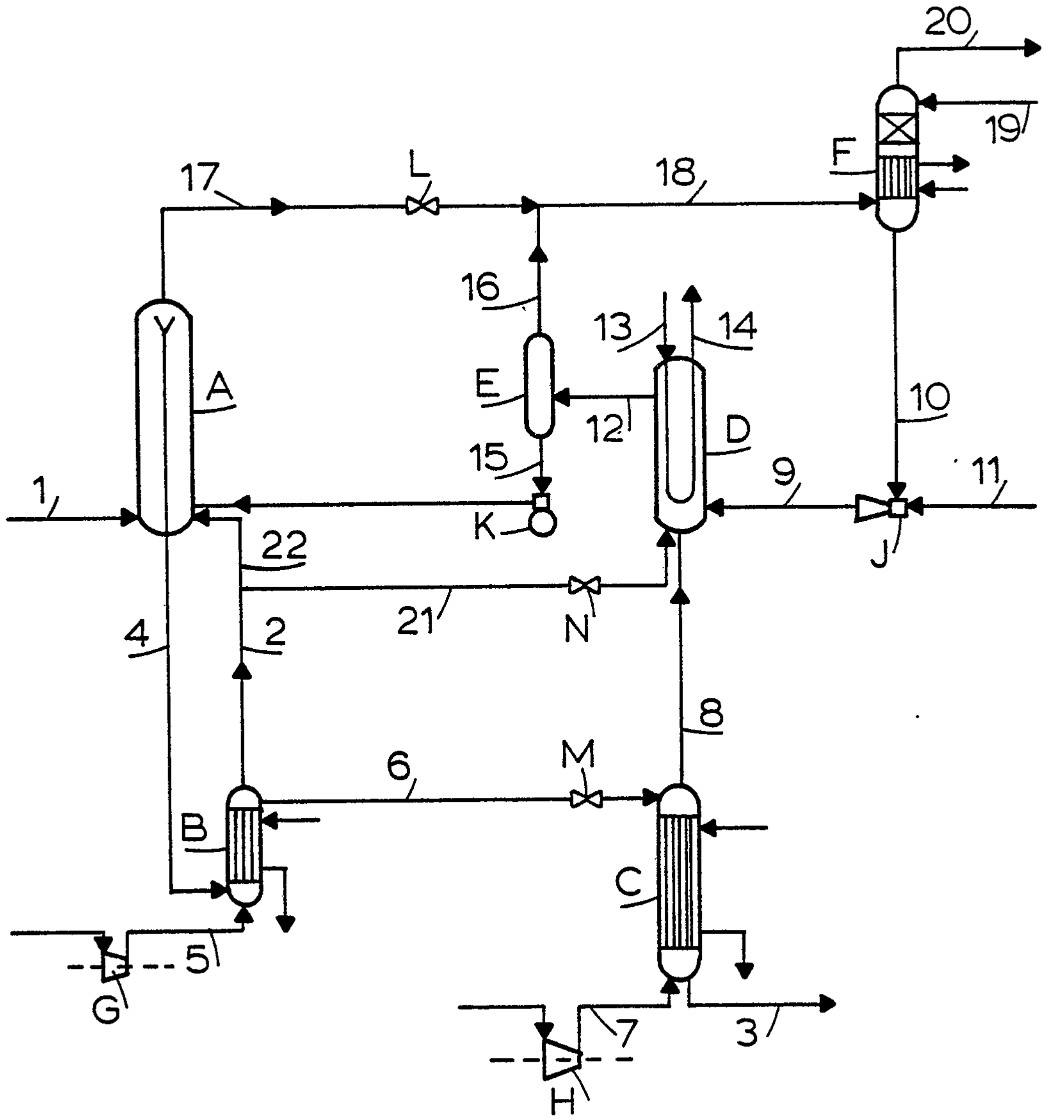
20
19
F
17
L
18
16
13
14
E
10
A
12
D
15
9
11
1
K
J
22
4
21
N
2
8
6
M
B
C
5
G
7
3
H

0157448

European Patent Office

# EUROPEAN SEARCH REPORT

Application number
EP 85 20 0386

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-3 527 799 (I. MAVROVIC) * The whole document * --- | 1-6 | C 07 C 126/02 |
| A | US-A-4 053 507 (INOUE et al.) * The whole document * --- | 1-6 | |
| D,A | GB-A-2 107 311 (AMMONIA CASALE S.A.) * The whole document * --- | 1-6 | |
| D,A | GB-A-2 087 381 (SNAMPROGETTI S.p.A.) * The whole document * ----- | 1-6 | |
| | | | TECHNICAL FIELDS SEARCHED (Int. Cl.4) |
| | | | C 07 C 126/00 |

The present search report has been drawn up for all claims

| Place of search | Date of completion of the search | Examiner |
|---|---|---|
| THE HAGUE | 12-06-1985 | MAISONNEUVE J.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document